# EUROPEAN PATENT APPLICATION

(11) **EP 2 006 681 A1**
(43) Date of publication of application: **24.12.2008**
(21) Application number: 07737628.3
(22) Date of filing: 01.03.2007
(51) Int. Cl.: G01N 33/53, A61K 39/395, A61K 48/00, A61P 19/02, A61P 29/00

(54) **RHEUMATOID ARTHRITIS TEST METHOD AND TREATING METHOD**

(30) Priority: 02.03.2006 JP 2006056098
(71) Applicant: National University Corporation Chiba University, Chiba-shi Chiba, 2638522 (JP)
(72) Inventor: SUZUKI, Masahiko, Chiba-shi, Chiba 260-8670 (JP); AOSAI, Fumie, Chiba-shi, Chiba 260-8670 (JP); UENO, Koichi, Chiba-shi, Chiba 260-8670 (JP)
(74) Representative: Zwicker, Jörk
(86) International application number: PCT/JP2007/053948
(87) International publication number: WO 2007/100058

(57) **Abstract**

It is intended to evaluate the prognosis of rheumatoid arthritis based on the evaluation of the disease severity of the patient to select a treatment method suitable for each rheumatoid arthritis patients, and to measure HSC71 protein levels of the patients before and after the administrations of therapeutic agents to determine the efficacy of various therapeutic agents for each of rheumatoid arthritis patients, and to use thereof as a remedy for preventing onset and progression of rheumatoid arthritis. It has been revealed that whether HSC71 (heat shock cognate protein 71), which is a member of the heat shock protein 70 family, shows any difference in expression between rheumatoid arthritis patients and healthy subjects. In the case where the HSC71 protein is accelerated in a rheumatoid arthritis patient, the HSC71 concentrations in the serum, synovial fluid, serebrospinal fluid, urine, saliva and sputum of the patient are measured to thereby examine the disease severity of rheumatoid arthritis. Based thereon, the prognosis of the rheumatoid arthritis is evaluated and a method suitable for the patient is selected. HSC71 protein levels are measured before and after the administration of therapeutic agents to a rheumatoid arthritis patient and the efficacy of the therapeutic agents are determined. Further, Use as a remedy or preventing the onset and progression of rheumatoid arthritis is intended.

SEQUENCE LISTING

<110>  National University Corporation Chiba University

<120>  The test methods and treatments in patients with rheumatoid arthritis

<130>  535-8

<160>  2

<170>  PatentIn version 3.1

<210>  1
<211>  27
<212>  DNA
<213>  Artificial Sequence

<220>
<223>  Oligonucleotide forward primer used in RT-PCR for amplifying huma
       n HSC71

<400>  1
gggctcgaga tgtccaaggg acctgca                                          27


<210>  2
<211>  29
<212>  DNA
<213>  Artificial Sequence

<220>
<223>  Oligonucleotide reverse primer used in RT-PCR for amplifying huma
       n HSC71

<400>  2
ggggatccgg cttaatcaac ctcttcaat                                        29

## Description

### TECHNICAL FIELD

The present invention relates to using antibodies to HSC71 (heat shock cognate protein 71) which is one member of heat shock protein 71 family for measuring HSC71 protein level in serum synovial fluid, celebrospinal fluid, urine, saliva, sputum from rheumatoid arthritis patients to determine disease severity of rheumatoid arthritis, and evaluating thereby prognosis of rheumatoid arthritis to select a treatment method suitable for each of the patients, and measuring HSC71 protein levels before and after administrations of therapeutic agents to rheumatoid arthritis patients to evaluate efficacy of each kind of therapeutic agents in each of the patients, and using as a therapeutic agent to prevent onset and progression of rheumatoid arthritis.

### BACKGROUND ART

Rheumatoid arthritis includes chronic rheumatoid arthritis and malignant rheumatoid arthritis, and is estimated to have a high incidence rate in Japan. Rheumatoid arthritis is diagnosed using, for example, the American Rheumatoid Association diagnostic criteria by evaluating observations including morning stiffness, symmetric poly-arthritis, subcutaneous nodules, and X-ray image of hand joints. In spite of yearly increase of the number of the patients, efficient therapeutic agents and prevention methods have not yet been found out, though a novel medicament for preventing/treating rheumatoid arthritis has been proposed (Patent document 1).

The present inventors made a unique investigation which revealed that patients with myasthenia gravis (hereinafter referred to as MG) showed a significantly higher serum concentration of anti-HSC71 antibody than healthy subjects and patients with other autoimmune diseases, and have filed a patent application (Patent document 2). However, the expression of HSC71 protein has not been studied for rheumatoid arthritis patients, therefore the usefulness of the protein has not been known.
Patent document 1: Japanese Patent Application Laid Open No. 2003-026598
Patent document 2: Japanese Patent Application Laid Open No. 2004-257862

### DISCLOSURE OF THE INVENTION

### [PROBLEM TO BE SOLVED BY THE INVENTION]

An object of the present invention in order to solve the above problem is to determine disease severity of rheumatoid arthritis patients and thereby to evaluate the prognosis of rheumatoid arthritis to select a treatment method suitable for each of the patients; to measure the HSC71 protein levels in a body fluid before and after administrations of therapeutic agents to rheumatoid arthritis patients to evaluate efficacy of each kind of the therapeutic agents; and use as a therapeutic agent for preventing onset and progression of rheumatoid arthritis.

### [MEANS FOR SOLVING THE PROBLEM]

The present inventors have studied with great effort and revealed that whether HSC71 shows any difference in expression between rheumatoid arthritis patients and healthy subjects.

In case of rheumatoid arthritis patients with increased expression of HSC71 protein, HSC71 protein concentration in the serum, synovial fluid, cerebrospinal fluid, urine, saliva, sputum of the patients is measured for determining the disease severity of rheumatoid arthritis.

The above determination can be used as a basis for evaluating prognosis of rheumatoid arthritis to select a treatment method suitable for each of the patients. The HSC71 protein levels are determined in rheumatoid arthritis patients before and after administrations of various therapeutic agents to evaluate efficacy of each kind of therapeutic agents for each of the patients, and to use as a therapeutic agent to prevent the onset and progression of rheumatoid arthritis. The present invention comprises followings.

The present invention according to claim 1 is a diagnostic method of rheumatoid arthritis, comprising measuring HSC71 protein concentration in a sample derived from a living body by using an anti-HSC71 antibody and determining disease severity of the rheumatoid arthritis.

The present invention according to claim 2 is a treatment method of rheumatoid arthritis, comprising evaluating prognosis of rheumatoid arthritis by measuring HSC71 protein level in each patient, and determining a treatment method suitable for each patient.

The present invention according to claim 3 is a treatment method of rheumatoid arthritis, comprising measuring HSC71 protein levels in a rheumatoid arthritis patient before and after administrations of therapeutic agents, and evaluating efficacy of each kind of therapeutic agents for the patient.

The present invention according to claim 4 is a treatment method of rheumatoid arthritis, comprising inhibiting the function of HSC 71 protein in a rheumatoid arthritis patient by using an antibody to HSC71 protein, and thereby preventing progression of the rheumatoid arthritis.

The present invention according to claim 5 is a treatment method of rheumatoid arthritis, comprising inhibiting the function of HSC 71 protein in an subject with possible onset of rheumatoid arthritis by using an antibody to HSC71 protein, and thereby preventing onset of rheumatoid arthritis.

The present invention according to claim 6 is a treatment method of rheumatoid arthritis, comprising inhibiting expression of HSC 71 protein at a DNA and RNA level in a rheumatoid arthritis patient, and thereby preventing progression of the rheumatoid arthritis.

The present invention according to claim 7 is a treatment method of rheumatoid arthritis, comprising inhibiting expression of HSC 71 protein at a DNA and RNA level in an subject with possible onset of rheumatoid arthritis, and thereby preventing onset of rheumatoid arthritis.

### [ADVANTAGE OF THE INVENTION]

Aosai, one of the present inventors, has prepared an antibody to the HSC71 by administering recombinant human HSC71 and a Freund adjuvant to BALB/c mice by intra-peritoneal injection and culturing excised spleen cells. The antibody have allowed application of an ELISA method for measuring HSC71 protein in serum, synovial fluid, cerebrospinal fluid, urine, saliva, sputum from human beings. This system allowed elucidation of a significant difference in expression of the protein between rheumatoid arthritis patients and healthy subjects, and thereby revealed the relationship between HSC71 and disease severity of rheumatoid arthritis patients.
These findings have allowed determination of disease severity of rheumatoid arthritis by utilizing HSC71; thereby evaluating prognosis of the rheumatoid arthritis to select a treatment method suitable for each of the patients; measuring HSC71 protein levels in a body fluid before and after administrations of various therapeutic agents to evaluate efficacy of each kind of therapeutic agents for each of the patients; and using as a remedy to prevent the onset and progression of rheumatoid arthritis.

### DETAILED DESCRIPTION

The embodiments of the present invention (hereinafter referred to as the present invention) are described below in more details with reference to Examples. The present invention is not limited to these embodiments.
A method of the present invention measures an anti-HSC71 antibody in a body fluid collected from a living body. HSC71 is a member of heat shock proteins 70 (HSP70) that are present in wide variety of species including a human being, and the amino acid sequence thereof is known as well as the nucleotide sequence of a gene encoding thereof. For example, the amino acid sequence of human HSC71 and the nucleotide sequence of a gene encoding thereof are disclosed as GenBank accession No.Y00371.

The test method of the present invention is normally used for human, as the following Example uses a human serum. However, the method is applicable to the other species than a human being because the amino acid sequence of HSC71 is well conserved in wide variety of species. Preferable examples of a body fluid include blood, and its component such as serum and plasma.

An anti-HSC71 antibody in a body fluid can be measured by a well known immunoassay. A substance used in the immunoassay which reacts with the anti-HSC71 antibody by the antigen-antibody reaction may be a native HSC71, and is preferably a recombinant HSC71 prepared by genetic engineering techniques because of its easy production. The substance may be a part of the recombinant HSC71 or may contain any other component than HSC71, as long as the substance can react with the anti-HSC71 antibody by the antigen-antibody reaction. Further, HSC71 that is modified with substitution, deletion, or insertion of a small number of amino acids in the amino acid sequence, preferably less than several amino acids, may also be used, as long as it can react with the anti-HSC71 antibody by the antigen-antibody reaction. Needless to say, it is preferable to use the HSC71 derived from an animal to be tested as an antigen (for example, the human HSC71 is used to test for human). The recombinant HSC71 can be easily prepared by a conventional method, since the amino acid sequence and the nucleotide sequence of a gene encoding thereof are known as described above. The Example described later specifically discloses a method for producing human HSC71 by genetic engineering techniques using Escherichia coli as a host as well.

Immunoassay itself is well known, and any known immunoassay can be used. Immunoassays may be classified into sandwich assay, competition assay, agglutination assay, and so on based on reaction-type, while they may be classified into enzyme immunoassay, radiation immunoassay, fluorescence immunoassay, and so on based on labeling substances. The present invention can use any of these assays, and preferably uses the sandwich assay because of its simple procedure and accurate measurement. The present invention particularly preferably uses, but is not limited to, an enzyme linked immunosorbent assay (ELISA) because it is safe and needs no specific apparatus.

The present invention also provides an agent for testing for rheumatoid arthritis, comprising a substance which reacts with the anti-HSC71 antibody by the antigen-antibody reaction. The substance which reacts with the anti-HSC71 antibody by the antigen-antibody reaction is as described above, and is used in the aforementioned immunoassay as an antigen or a hapten that reacts with the anti-HSC71 antibody by the antigen-antibody reaction.

### MATERIALS AND METHODS

Sera from 156 patients with rheumatoid arthritis who meet the American Rheumatoid Association diagnostic criteria 1987 were used as well as sera from 36 healthy persons. All the sera were kept at -30 °C.

### Cloning of human HSC71

Total RNA of P36 cells, a human melanoma cell line, was prepared by a single process guanidium isothiocyanate-phenol-chloroform extraction method (TRIzol (trade name), made by GIBCO BRL, Gaithersburg, Maryland, USA). Oligonucleotide102030405 primers were designed so as to link the genomic human HSC71 DNA sequence (GenBank accession No. Y00371) and the neighboring region to restriction enzyme sites (the restriction enzyme names: XhoI and BamHI) suitable for cloning. cDNA preparation and PCR for amplifying human HSC71 were carried out using Takara RNA kit with AMV RTase (Takara Shuzo). PCR primers used were as follows: the sense primer: 5'-gggctcgagatgtccaagggacctgca-3'; and the anti-sense primer: 5'-ggggatccggcttaatcaacctcttcaat-3'. PCR was carried out by repeating the cycle up to 36 times that comprises a denaturing process at 94°C for 1 min, an annealing process at 55°C for 2 min, and an elongating process at 72°C for 2 min. The PCR product thus obtained was inserted into the cloning site of the pBluescript II SK (trade name) phagemid vector for cloning. The nucleotide sequence of the inserted PCR product in the recombinant vector thus obtained was determined by the double strand dideoxy-sequencing method using the ABI DNA sequencer (ABI Applied Biosystems Division, Foster, California, USA).

### Expression of a recombinant human HSC71

In order to synthesize a recombinant human HSC71, HSC71 cDNA was excised from the recombinant pBluescript II SK (trade name) by using the restriction enzymes XhoI and BamHI, and inserted into a vector which is the expression vector pET-15b (Novagen, USA) modified by introducing an another XhoI cleavage site into the cloning site to allow insertion of an insert DNA having an XhoI site at the N-terminal and a BamHI site at the C terminal. Escherichia coli BL21 (DE) was transformed with the obtained recombinant vector, and supplied with 1mM isopropyl β-D-thiogalactopyranoside (IPTG) (Katayama Chemicals) to induce the synthesis of the recombinant protein. The recombinant 6 X His-HSC71 protein (comprising an amino acid sequence derived from the expression vector pET-15b that is Met-Gly-Ser-Ser at the N-terminal followed by 6 histidines, further followed by Ser-Ser-Gly-Leu-Val-Pro-Arg-Gly-Ser-His-Met, and then followed by the amino acid sequence of HSC71) was purified from the cell extract by nickel chelate affinity chromatography that utilizes the binding property of the 6 histidines to nickel according to the Novagen's instruction. The crude recombinant HSC71 purified by the nickel chelate affinity chromatography was analyzed by SDS-PAGE, which showed the molecular weight to be 71kDa.

### Measurement of anti-HSC71 antibody by ELISA

The recombinant human HSC71 solution at a concentration of 10 µg/ml in PBS was used for coating a flat bottom microtiter plate. After over night incubation, non-specific binding sites were blocked with a PBS containing 0.05% Tween 20 (trade name), 1mM EDTA, 0.25% BSA, and 0.05% NaN₃. The patient serum and the control serum both diluted at 1:200 were added to each of the wells and incubated at room temperature for 2 hours. After washing the plate, an alkali phosphatase-conjugated goat anti-human IgG antibody (Sigma-Aldrich) diluted at 1:1000 was added and incubated at room temperature for 2 hours. For colorization, the wells were washed and added with a p-nitrophenyl phosphate substrate solution, and the absorption was measured at a wavelength of 405nm.

### EXAMPLE

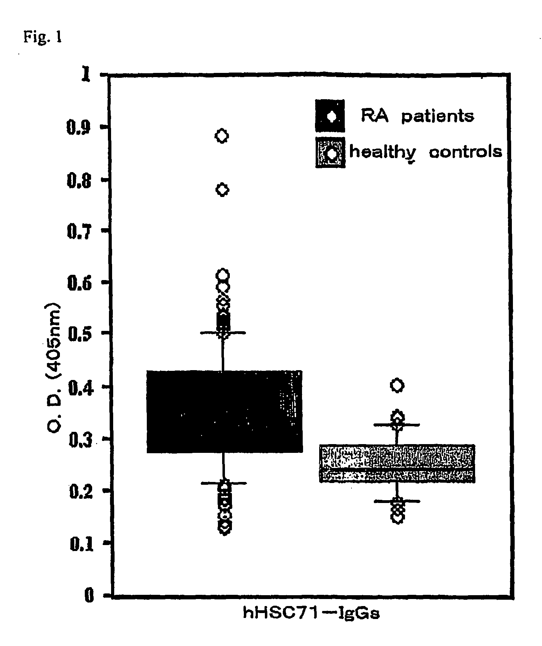

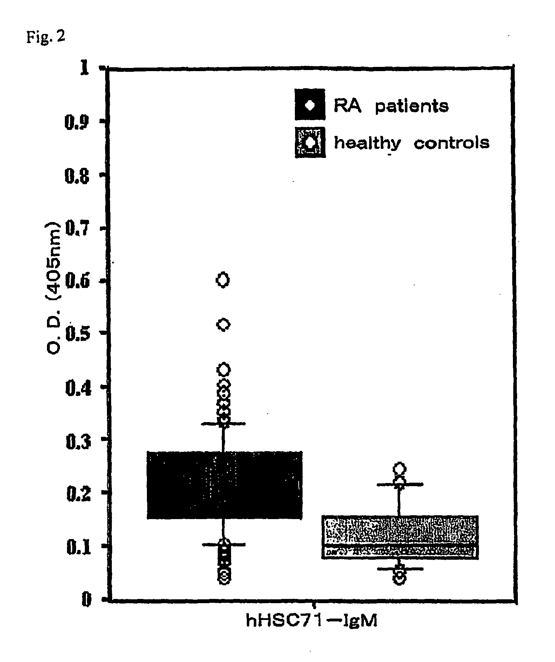

The sera from 156 patients with rheumatoid arthritis and 36 healthy subjects were used to measure HSC71-IgG antibody titers (Fig. 1) and HSC71-IgM antibody titers (Fig. 2). The rheumatoid arthritis patients had met the American Rheumatoid Association diagnostic criteria 1987. The rheumatoid arthritis patients showed significantly high antibody titers. The present invention is further described below.

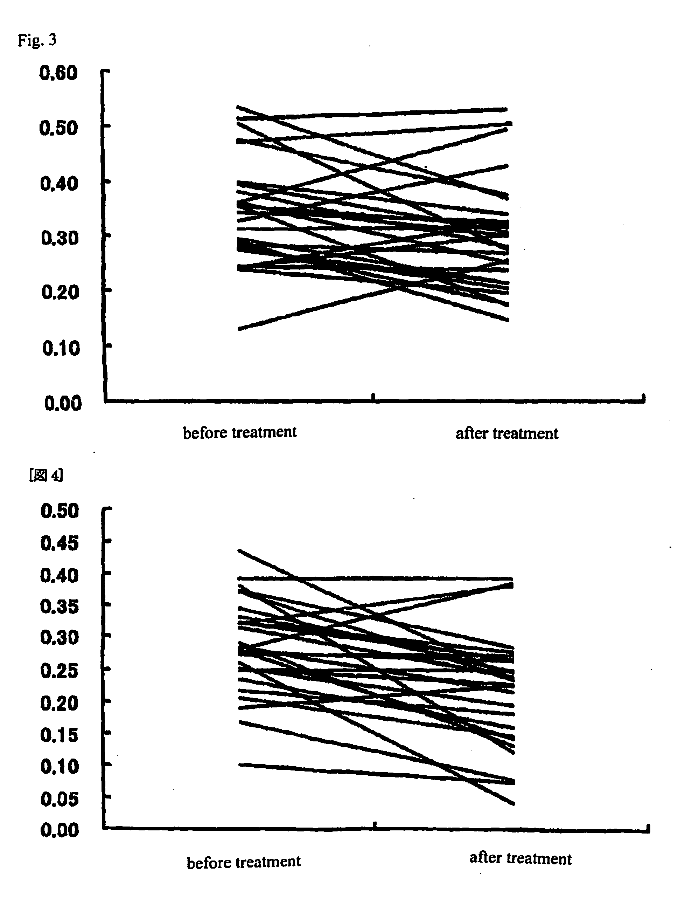

27 patients with rheumatoid arthritis were treated with an anti-rheumatic drug such as methotrexate, salazosulfapyridine, and bucillamine, a steroidal drug, and a biological drug. The sera collected before the treatment and 3 months or more after the treatment were used to measure HSC71-IgG antibody titers (Fig. 3) and HSC71-IgM antibody titers (Fig. 4). After the treatment, these cases showed improvement in clinical symptoms accompanied by a general inclination of decrease in the HSC71-IgG antibody titers and the HSC71-IgM antibody titers that were statistically significant. An average CRP of the patients before the treatment was 2.56 mg/dl, while that of the patients after the treatment was 0.36 mg/dl. There were observed several cases which showed no decrease or rather increase in the antibody titers.

### (Result)

As described above, according to the present invention, it is possible to measure HSC71 protein concentration in serum, synovial fluid, cerebrospinal fluid, urine, saliva, sputum from rheumatoid arthritis patients using the antibody to HSC71, and to determine the disease severity of rheumatoid arthritis, and thereby to evaluate the prognosis of the rheumatoid arthritis to select a treatment method suitable for each of the patients.

Further, according to the present invention, it is possible to measure HSC71 protein levels in rheumatoid arthritis patients before and after administrations of various therapeutic agents, and thereby to evaluate efficacy of each of the therapeutic agents for each of the patients.

Furthermore, according to the present invention, it is possible to use as a remedy to prevent the onset and progression of rheumatoid arthritis.

### INDUSTRIAL APPLICABILITY

The present invention provides highly efficient methods for diagnosis, treatment, and prevention of rheumatoid arthritis which have been difficult hitherto.

### BRIEF DESCRIPTION OF THE DRAWINGS

Fig. 1 shows HSC71-IgG antibody titers in 156 patients with rheumatoid arthritis and 36 healthy subjects.
Fig. 2 shows HSC71-IgM antibody titers.
Fig. 3 shows a change in HSC71-IgG antibody titers before and after treatment.
Fig. 4 shows a change in HSC71-IgM antibody titers before and after treatment.

## Claims

1. A method of testing for rheumatoid arthritis, comprising measuring HSC71 protein concentration in a sample obtained from a living body by using an anti-HSC71 antibody and determining disease severity of rheumatoid arthritis.

2. A method of treating rheumatoid arthritis, comprising evaluating prognosis of rheumatoid arthritis by measuring HSC71 protein level in each patient, and determining a treatment method suitable for the patient.

3. A method of treating rheumatoid arthritis, comprising measuring HSC protein levels in a rheumatoid arthritis patient before and after administrations of various therapeutic agents, and thereby evaluating efficacy of each kind of the therapeutic agents for the patient.

4. A method of treating rheumatoid arthritis, comprising inhibiting HSC71 protein from expressing the function in a rheumatoid arthritis patient by using an antibody to HSC71 protein, and thereby preventing progression of the rheumatoid arthritis.

5. A method of treating rheumatoid arthritis, comprising inhibiting HSC71 protein from expressing the function in a subject with possible onset of rheumatoid arthritis by using an antibody to HSC71 protein, and thereby preventing onset of rheumatoid arthritis.

6. A method of treating rheumatoid arthritis, comprising inhibiting expression of HSC71 protein at a DNA and RNA level in a rheumatoid arthritis patient, and thereby preventing progression of the rheumatoid arthritis.

7. A method of treating rheumatoid arthritis, comprising inhibiting expression of HSC71 protein at a DNA and RNA level in a subject with possible onset of rheumatoid arthritis, and thereby preventing onset of rheumatoid arthritis.
